# EUROPÄISCHE PATENTSCHRIFT

(11) **EP 0 632 004 B1**
(45) Veröffentlichungstag und Bekanntmachung des Hinweises auf die Patenterteilung: **06.11.1996**
(21) Anmeldenummer: 94109477.3
(22) Anmeldetag: 20.06.1994
(51) Int. Cl.: C07C 45/00, C07C 49/395, C07C 49/403, C07C 49/413

(54) **Verfahren zur Herstellung von cyclischen Ketonen**
Process for the preparation of cyclic ketones
Procédé pour la préparation de cétones cycliques

(30) Priorität: 30.06.1993 DE 4321692
(43) Veröffentlichungstag der Anmeldung: 04.01.1995
(73) Patentinhaber: BASF Aktiengesellschaft, 67063 Ludwigshafen (DE)
(72) Erfinder: Schroeder, Juergen, Dr., D-67071 Ludwigshafen (DE); Ebel, Klaus, Dr., D-67067 Ludwigshafen (DE); Schommer, Charles, Dr., D-67063 Ludwigshafen (DE)

(56) Entgegenhaltungen:
- EP-A- 0 251 111
- DE-C- 591 269

## Beschreibung

Die vorliegende Erfindung betrifft ein Verfahren zur Herstellung von cyclischen Ketonen durch Umsetzung mit aliphatischen Dicarbonsäuredinitrilen bei erhöhten Temperaturen an festen oxidischen Katalysatoren.

Aus der EP-A-251 111 ist ein Verfahren zur Herstellung von cyclischen Ketonen aus aliphatischen Dicarbonsäureestern an oxidischen Katalysatoren bei Temperaturen von 300 bis 345°C bekannt. Bei der Herstellung von Cycloheptanon werden nur moderate Ausbeuten erzielt. Verbesserungsfähig bei dem Verfahren ist außerdem, daß die Dicarbonsäureester erst aus den besser verfügbaren Dicarbonsäuren bzw. Dicarbonsäuredinitrilen hergestellt werden müssen.

Der vorliegenden Erfindung lag daher die Aufgabe zugrunde, den zuvor genannten Nachteilen abzuhelfen.

Demgemäß wurde ein neues und verbessertes Verfahren zur Herstellung von cyclischen Ketonen der allgemeinen Formel I 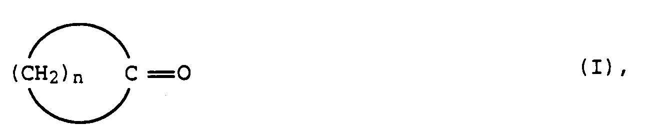 in der n eine ganze Zahl von 4 bis 6 bedeutet, gefunden, welches dadurch gekennzeichnet ist, daß man aliphatische Dicarbonsäuredinitrile der allgemeinen Formel II

NC-(CH₂)ₙ-CN (II),

in der n die obengenannte Bedeutung hat, in der Gasphase in Gegenwart von Wasser bei Temperaturen von 250 bis 500°C an festen oxidischen Katalysatoren umsetzt.

Das erfindungsgemäße Verfahren läßt sich wie folgt durchführen:

Ein Gemisch aus Dicarbonsäuredinitril II und Wasser kann bevorzugt in der Gasphase über den auf Reaktionstemperatur von 250 bis 500°C, bevorzugt 300 bis 400°C erhitzten Katalysator geleitet werden. Der Reaktionsdruck kann in weiten Grenzen variiert werden und beträgt in der Regel 0,01 bis 50 bar, bevorzugt 0,1 bis 5 bar, besonders bevorzugt Normaldruck (Atmosphärendruck). Gegebenenfalls können auch unter Reaktionsbedingungen inerte Gase, wie Stickstoff oder Argon, zugeführt werden. Das den Reaktor verlassende Gasgemisch kann kondensiert, die organische Phase abgetrennt und fraktioniert destilliert werden.

Als Ausgangsverbindung II eignen sich Adipinsäuredinitril, Pimelinsäuredinitril und Korksäuredinitril.

Als Katalysatoren eignen sich bevorzugt feste oxidische Katalysatoren Oxide der III. und/oder IV. Hauptgruppe und/oder der IV. Nebengruppe des Periodensystems der Elemente und/oder des Zinks wie Boroxid, Aluminiumoxid, Galliumoxid, Siliciumdioxid, Germaniumdioxid, Zinndioxid, Titandioxid, Zirkoniumdioxid, Hafniumdioxid und Zinkoxid, bevorzugt Aluminiumoxid, Siliciumdioxid, Zinkoxid und Titandioxid sowie deren Gemische. Ganz besonders gut geeignet sind Gemische aus Aluminiumoxid oder Siliciumdioxid und Titandioxid.

Es ist zwar möglich, die erfindungsgemäße Umsetzung mit stöchiometrischem Zusatz von Wasser durchzuführen, allerdings wird durch einen Wasserüberschuß eine bemerkenswerte Erhöhung von Selektivität und Standzeit erreicht. Man arbeitet in der Regel im Molverhältnis von Wasser zum Dicarbonsäuredinitril II von 3 : 1 bis 200 : 1, bevorzugt 4 : 1 bis 50 : 1, besonders bevorzugt 5 : 1 bis 30 : 1.

Die Umsetzung kann diskontinuierlich oder kontinuierlich in einem Festbettreaktor, in Sumpf- oder Rieselfahrweise, oder in einem Wirbelbettreaktor durchgeführt werden.

Im Vergleich zu den bekannten Verfahren liefert das erfindungsgemäße Verfahren auf einfacherem und wirtschaftlicherem Wege cyclische Ketone.

Die nach dem erfindungsgemäßen Verfahren erhältlichen cyclischen Ketone sind wertvolle Zwischenprodukte für die Synthese von Pflanzenschutzmitteln und Pharmazeutika.

### Beispiele

### Beispiel 1

Stündlich werden 40,8 g Korksäuredinitril und 97,2 g Wasser verdampft und mit 90 Nl/h Stickstoff durch einen auf 360°C erhitzten Wirbelbettreaktor geleitet. Der Wirbelbettreaktor ist mit 500 ml eines Katalysators aus 75 Gew.-% Titandioxid und 25 Gew.-% gamma-Aluminiumoxid gefüllt. Die Reaktionsgase werden anschließend kondensiert und die Phasen getrennt. Man erhält stündlich 28,5 g Cycloheptanon (85 % der Theorie bezogen auf eingesetztes Korksäuredinitril).

### Beispiel 2

Stündlich werden 36,6 g Pimelinsäuredinitril und 97,2 g Wasser verdampft und mit 90 Nl/h Stickstoff durch einen auf 360°C erhitzten Wirbelbettreaktor geleitet. Der Wirbelbettreaktor ist mit 500 ml eines Katalysators aus 75 Gew.-% Titandioxid und 25 Gew.-% gamma-Aluminiumoxid gefüllt. Die Reaktionsgase werden anschließend kondensiert und die Phasen getrennt. Man erhält stündlich 7,9 g Cyclohexanon (27 % der Theorie bezogen auf eingesetztes Pimelinsäuredinitril).

### Beispiel 3

Stündlich werden 32,4 g Adipinsäuredinitril und 97,2 g Wasser verdampft und mit 90 Nl/h Stickstoff durch einen auf 300°C erhitzten Wirbelbettreaktor geleitet. Der Wirbelbettreaktor ist mit 500 ml eines Katalysators aus 75 Gew.-% Titandioxid und 25 Gew.-% gamma-Aluminiumoxid gefüllt. Die Reaktionsgase werden anschließend kondensiert und die Phasen getrennt. Man erhält stündlich 18,1 g Cyclopentanon (72 % der Theorie bezogen auf eingesetztes Adipinsäuredinitril).

### Beispiel 4

Stündlich werden 32,4 g Adipinsäuredinitril und 97,2 g Wasser verdampft und mit 90 Nl/h Stickstoff durch einen auf 300°C erhitzten Wirbelbettreaktor geleitet. Der Wirbelbettreaktor ist mit 500 ml eines Katalysators aus 75 Gew.-% Titandioxid und 25 Gew.-% Siliciumdioxid gefüllt. Die Reaktionsgase werden anschließend kondensiert und die Phasen getrennt. Man erhält stündlich 11,6 g Cyclopentanon (46 % der Theorie bezogen auf eingesetztes Adipinsäuredinitril).

### Beispiel 5

Stündlich werden 40,8 g Korksäuredinitril und 64,8 g Wasser verdampft und mit 170 Nl/h Stickstoff durch einen auf 360°C erhitzten Wirbelbettreaktor geleitet. Der Wirbelbettreaktor ist mit 500 ml eines Katalysators aus 75 Gew.-% Titandioxid und 25 Gew.-% gamma-Aluminiumoxid gefüllt. Die Reaktionsgase werden anschließend kondensiert und die Phasen getrennt. Man erhält stündlich 25,1 g Cycloheptanon (75 % der Theorie bezogen auf eingesetztes Korksäuredinitril).

## Patentansprüche

1. Verfahren zur Herstellung von cyclischen Ketonen der allgemeinen Formel I 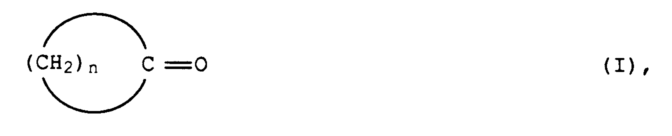 in der n eine ganze Zahl von 4 bis 6 bedeutet, dadurch gekennzeichnet, daß man aliphatische Dicarbonsäuredinitrile der allgemeinen Formel II
NC-(CH₂)ₙ-CN (II),
in der n die obengenannte Bedeutung hat, in der Gasphase in Gegenwart von Wasser bei Temperaturen von 250 bis 500°C an festen oxidischen Katalysatoren umsetzt.

2. Verfahren zur Herstellung von cyclischen Ketonen I nach Anspruch 1, dadurch gekennzeichnet, daß man als feste oxidische Katalysatoren Oxide der III. und/oder IV. Hauptgruppe und/oder der IV. Nebengruppe des Periodensystems der Elemente und/oder des Zinks einsetzt.

3. Verfahren zur Herstellung von cyclischen Ketonen I nach Anspruch 1, dadurch gekennzeichnet, daß man als feste oxidische Katalysatoren Aluminiumoxid, Siliciumdioxid, Zinkoxid und/oder Titandioxid einsetzt.

4. Verfahren zur Herstellung von cyclischen Ketonen I nach Anspruch 1, dadurch gekennzeichnet, daß man die Umsetzung bei Temperaturen von 300 bis 400°C durchführt.

5. Verfahren zur Herstellung von cyclischen Ketonen I nach Anspruch 1, dadurch gekennzeichnet, daß man Wasser und die aliphatischen Dicarbonsäuredinitrile II im Molverhältnis von 3 : 1 bis 200 : 1 einsetzt.

6. Verfahren zur Herstellung von cyclischen Ketonen I nach Anspruch 1, dadurch gekennzeichnet, daß man Wasser und die aliphatischen Dicarbonsäuredinitrile II im Molverhältnis von 4 : 1 bis 50 : 1 einsetzt.

7. Verfahren zur Herstellung von cyclischen Ketonen I nach Anspruch 1, dadurch gekennzeichnet, daß man Wasser und die aliphatischen Dicarbonsäuredinitrile II im Molverhältnis von 5 : 1 bis 30 : 1 einsetzt.

## Claims

1. A process for preparing cyclic ketones of the general formula I 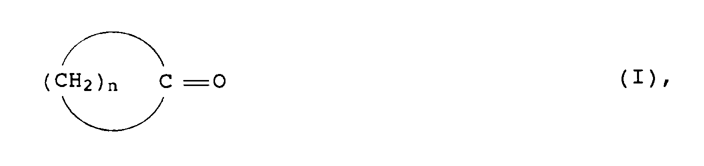 where n is an integer from 4 to 6, which comprises reacting aliphatic dicarbonitriles of the general formula II
NC-(CH₂)ₙ-CN (II),
where n has the abovementioned meaning, in the gas phase in the presence of water at from 250 to 500°C on solid oxide catalysts.

2. A process for preparing cyclic ketones I as claimed in claim 1, wherein oxides of group IIIa and/or IVa and/or IVb of the periodic table of the elements and/or of zinc are employed as solid oxide catalysts.

3. A process for preparing cyclic ketones I as claimed in claim 1, wherein aluminum oxide, silicon dioxide, zinc oxide and/or titanium dioxide are employed as solid oxide catalysts.

4. A process for preparing cyclic ketones I as claimed in claim 1, wherein the reaction is carried out at from 300 to 400°C.

5. A process for preparing cyclic ketones I as claimed in claim 1, wherein water and the aliphatic dicarbonitriles II are employed in the molar ratio of from 3:1 to 200:1.

6. A process for preparing cyclic ketones I as claimed in claim 1, wherein water and the aliphatic dicarbonitriles II are employed in the molar ratio of from 4:1 to 50:1.

7. A process for preparing cyclic ketones I as claimed in claim 1, wherein water and the aliphatic dicarbonitriles II are employed in the molar ratio of from 5:1 to 30:1.

## Revendications

1. Procédé de préparation de cétones cycliques de formule générale I 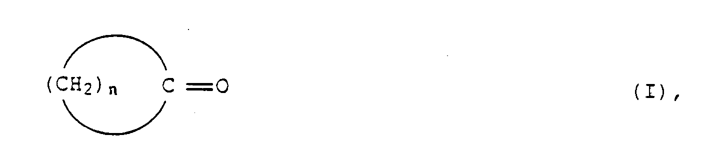 dans laquelle n est un nombre entier de 4 à 6, caractérisé en ce l'on fait réagir des dinitriles d'acides dicarboxyliques aliphatiques de formule générale II
NC-(CH₂)ₙ-CN (II)
dans laquelle n a la signification donnée ci-dessus, en phase gazeuse, en présence d'eau, à des températures de 250 à 500°C et en présence de catalyseurs solides de type oxyde.

2. Procédé de préparation de cétones cycliques I selon la revendication 1, caractérisé en ce que l'on utilise, comme catalyseurs solides de type oxyde, des oxydes des éléments des groupes IIIA et/ou IVA et/ou du groupe IVB de la classification périodique et/ou du zinc.

3. Procédé de préparation de cétones cycliques I selon la revendication 1, caractérisé en ce que l'on utilise, comme catalyseurs solides de type oxyde, de l'oxyde d'aluminium, du dioxyde de silicium, de l'oxyde de zinc et/ou du dioxyde de titane.

4. Procédé de préparation de cétones cycliques I selon la revendication 1, caractérisé en ce que l'on conduit la réaction à des températures de 300 à 400°C.

5. Procédé de préparation de cétones cycliques I selon la revendication 1, caractérisé en ce que l'on met en réaction l'eau et les dinitriles d'acides dicarboxyliques aliphatiques II dans un rapport molaire de 3:1 à 200:1.

6. Procédé de préparation de cétones cycliques I selon la revendication 1, caractérisé en ce que l'on met en réaction l'eau et les dinitriles d'acides dicarboxyliques aliphatiques II dans un rapport molaire de 4:1 à 50:1.

7. Procédé de préparation de cétones cycliques I selon la revendication 1, caractérisé en ce que l'on met en réaction l'eau et les dinitriles d'acides dicarboxyliques aliphatiques II dans un rapport molaire de 5:1 à 30:1.
